(19) Europäisches Patentamt
European Patent Office
Office européen des brevets

(11) Veröffentlichungsnummer: **0 394 854 A1**

(12) **EUROPÄISCHE PATENTANMELDUNG**

(21) Anmeldenummer: **90107492.2**

(51) Int. Cl.5: **C07D 263/12, C07D 263/14**

(22) Anmeldetag: **19.04.90**

(30) Priorität: **28.04.89 DE 3914159**

(43) Veröffentlichungstag der Anmeldung: **31.10.90 Patentblatt 90/44**

(84) Benannte Vertragsstaaten: **GR**

(71) Anmelder: **Henkel Kommanditgesellschaft auf Aktien**
**Henkelstrasse 67**
**D-4000 Düsseldorf 13(DE)**

(72) Erfinder: **Falbe, Jürgen, Prof., Dr.**
**Linnéplatz 19**
**D-4040 Neuss 1(DE)**
Erfinder: **Fristad, William E., Dr.**
**Bockumer Strasse 165**
**D-4000 Düsseldorf 31(DE)**
Erfinder: **Krause, Horst Jürgen, Dr.**
**Am Nettchesfeld 22**
**D-4000 Düsseldorf(DE)**
Erfinder: **Neumann, Peter**
**Nosthoffenstrasse 61**
**D-4000 Düsseldorf(DE)**

(54) **Verfahren zur Herstellung von 2-Alkyl- bzw. Alkenyl-2-oxazolinen aus Fettsäureglyceriden.**

(57) Ein Verfahren zur Herstellung von 2-Alkyl- bzw. Alkenyl-2-oxazolinen, in denen die Alkyl- bzw. Alkenylgruppe ein gegebenenfalls hydroxy-substituierter Kohlenwasserstoffrest mit mindestens 7 Kohlenstoffatomen ist, ergibt die Titelverbindungen in hohen Ausbeuten, wenn man Glyceride von Fettsäuren mit mindestens 8 Kohlenstoffatomen mit 2-Aminoethanol in Gegenwart von Titan- bzw. Zirkoniumverbindungen des Typs $M(OR^2)_4$ (M = Ti bzw. Zr) umsetzt.

EP 0 394 854 A1

Xerox Copy Centre

## Verfahren zur Herstellung von 2-Alkyl- bzw. Alkenyl-2-oxazolinen aus Fettsäureglyceriden.

Die Erfindung betrifft ein Verfahren zur Herstellung von 2-Alkyl- bzw. Alkenyl-2-oxazolinen der allgemeinen Formel I

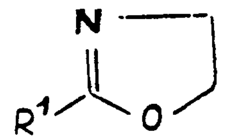

(I)

in der
$R^1$ ein gegebenenfalls hydroxy-substituierter Kohlenwasserstoffrest mit mindestens 7, insbesondere 7 bis 21 Kohlenstoffatomen in der Kohlenwasserstoffkette ist, durch Kondensation von Fettsäureethanolamiden oder von Fettsäureglyceriden und 2-Aminoethanol als deren Vorläufer in Gegenwart von Katalysatoren in flüssiger Phase.

2-Alkylsubstituierte 2-Oxazoline sind wertvolle Produkte, die unter anderem als Lösemittel oder Weichmacher und insbesondere als Polymerisationskomponenten eingesetzt werden. Zur Herstellung dieser Verbindungsklasse sind zahlreiche Verfahren beschrieben worden:

Die einfachste Herstellung ist die Cyclodehydratisierung von N-2-Hydroxyethyl-carbonsäureamiden (Chem. Rev. 44, 447 ff (1949), Chem. Rev. 71, 485 ff (1971)). Die Cyclisierung der unsubstituierten N-2-Hydroxyethyl-carbonsäureamide erfordert jedoch sehr drastische Bedingungen oder die Anwesenheit spezieller Katalysatoren. Während zur Herstellung leicht flüchtiger, kurzkettiger 2-Alkyl-2-oxazoline Reaktionen in der Gasphase in Gegenwart von dehydratisierend wirkenden Metalloxiden wie $Al_2O_3$, $SiO_2/Al_2O_3$, $Al_2O_3/TiO_2$, $TiO_2$ oder MgO sich als geeignet erwiesen haben, stellt man die schwerer flüchtigen, längerkettigen 2-Alkyl-2-oxazoline besser in flüssiger Phase her. Als Katalysatoren für die Flüssigphasenreaktion sind Verbindungen des Mangans, Kobalts, Molybdäns, Wolframs, Eisens, Cadmiums, Zinks und Zinns sowie der seltenen Erdmetalle beschrieben worden (vgl. US-PS 3 562 263, BE-PS 666 829, C.A. 87, 135353, C.A. 87, 135352, US-PS 3 681 329, US-PS 3 681 333, EP-OS 00 33 752, US-PS 4 543 414, US-PS 4 354 029, US-PS 4 443 611, EP-OS 0 105 944 und EP-OS 0 164 219). Die in den vorgenannten Veröffentlichungen beschriebenen Katalysatoren führen bei der Darstellung längerkettiger 2-Fettalkyl-2-oxazoline jedoch nicht zu guten Ausbeuten.

Es wurde nun gefunden, daß sich spezielle Titan- und Zirkoniumverbindungen ausgezeichnet als Katalysatoren in einem Verfahren der eingangs genannten Art eignen, wobei Ausbeuten bis ca. 85 % der Theorie, bezogen auf die Ausgangsverbindung, erzielt werden können.

Demgemäß ist die Erfindung auf ein Verfahren der eingangs genannten Art gerichtet, bei dem man Glyceride, insbesondere Triglyceride, von gegebenenfalls hydroxy-substituierten Fettsäuren mit mindestens 8, insbesondere 8 bis 22 Kohlenstoffatomen mit 2-Aminoethanol
in Gegenwart von Titan- bzw. Zirkoniumverbindungen der allgemeinen Formel II

$M(OR^2)_4$ (II)

in der M vierwertiges Titan oder Zirkonium und $R^2$ eine Alkylgruppe mit mindestens 2, insbesondere 2 bis 4 Kohlenstoffatomen, eine Acylgruppe mit mindestens 2, insbesondere 2 bis 10 Kohlenstoffatomen oder eine 2-Aminoethylenoxygruppe oder den Rest eines beta-Diketons der allgemeinen Formel III bedeuten,

$R^3$-C =CH-CO-$R^4$ (III)

in der $R^3$ und $R^4$ gleiche oder verschiedene Reste aus der von Alkylgruppen mit 1 bis 4 Kohlenstoffatomen und gegebenenfalls in p-Stellung substituiertem Phenyl gebildeten Gruppe sind, wobei jeweils zwei der Gruppen $R^2$ zusammen aus dem zweibindigen Rest eines zweiwertigen Alkohols mit 2 bis 4 Kohlenstoffatomen bestehen können,
in Gegenwart von Titanylacetylacetonat oder
in Gegenwart von Kondensationsprodukten von Titan(IV) bzw. Zirkonium(IV)-tetraalkoxylaten der allgemeinen Formel II, in der M und $R^2$ wie oben definiert sind, mit mehrfunktionellen Alkanolen, insbesondere mit 3 bis 12 Kohlenstoffatomen und 2 bis 6 Hydroxylgruppen,
kondensiert und die so erhaltenen 2-Alkyl- bzw. 2-Alkenyloxazoline unter Entfernung von gebildetem Wasser und Glycerin isoliert.

Das Verfahren der Erfindung ist auf sämtliche Fettsäureglyceride anwendbar, die von geradkettigen oder verzweigten, gesättigten oder ungesättigten Fettsäuren mit mindestens 8 Kohlenstoffatomen, insbesondere mit 8 bis 22 Kohlenstoffatomen, einschließlich hydroxy-substituierten Derivaten derselben, abgeleitet sind. Als Ausgangsverbindungen für das Verfahren der Erfindung bevorzugt sind Fettsäureglyceride, insbesondere Triglyceride, pflanzlichen, tierischen oder seetierischen Ursprungs einschließlich technischer Gemische und durch katalytische Hydrierung gehärtete Derivate derselben.

Typische Vertreter der in der vorstehend genannten Glyceriden auftretenden Fettsäuren sind Capryl-, Caprin-, Myristin-, Palmitin-, Stearin-, 12-Hydroxystearin-, Arachin-, Behen-, Lignocerin-, Laurolein-, Myristolein-, Palmitolein-, Öl-, Gadolein-, Eruca-, Ricinol-, Linol-, Linolen- und Arachidonsäure. Wie in der Fettchemie üblich, werden meist nicht die Fettsäureglyceride, sondern technische Gemische derselben eingesetzt, wie sie aus natürlichen Rohstoffen zugänglich sind, z.B. aus Lauricölen, Kokosöl, Palmöl, Palmkernöl, Sojaöl, Erdnußöl, Rüböl, Olivenöl, Leinöl, Sonnenblumenöl, Ricinusöl, Rindertalg, Schweineschmalz und Fischöl.

Die als Katalysatoren im Verfahren der Erfindung einsetzbaren Titan- bzw. Zirkoniumverbindungen sind bekannt und größtenteils im Handel erhältlich. Kondensationsprodukte von Titan(IV) bzw. Zirkonium(IV)-tetraalkoxylaten mit mehrfunktionellen Alkanolen mit 3 bis 12 Kohlenstoffatomen und 2 bis 6 Hydroxylgruppen wie Glycerin, Trimethylolpropan und Pentaerythrit sind Veresterungs- und/oder Umesterungskatalysatoren, die z.B. in der US-C 4,705,764, auf deren Inhalt hier bezug genommen wird, beschrieben sind. Ein geeignetes Polyalkanol ist weiterhin Polyvinylalkohol. Die ebenfalls als Katalysatoren geeigneten Titan- bzw. Zirkoniumtetra-(2-amino ethoxylate) sind aus Titan- bzw. Zirkoniumtetraalkoxylaten und 2-Aminoethanol herstellbar.

Gemäß einer bevorzugten Ausführungsform der Erfindung verwendet man als Katalysatoren Ester der Titansäure ($H_4TiO_4$) oder der Zirkonsäure ($H_4ZrO_4$) bzw. gemischte Anhydride der Titan-oder Zirkonsäure mit organischen Säuren der allgemeinen Formel II, in der M = Ti oder Zr und $R^2$ eine Alkylgruppe mit mindestens 2 oder mehr als 2, insbesondere 2 bis 4 Kohlenstoffatomen bzw. eine von einer Monocarbonsäure abgeleitete Acylgruppe mit 2 oder mehr als 2, insbesondere 2 bis 10 Kohlenstoffatomen bedeuten.

Gemäß einer weiteren vorteilhaften Ausführungsform der Erfindung verwendet man Katalysatoren aus der von Titan- bzw. Zirkonium-tetraethylat, -tetrapropylat, -tetraisopropylat, -tetrabutylat und -tetraacetat gebildeten Gruppe.

Gemäß einer weiteren vorteilhaften Ausführungsform der Erfindung verwendet man als Katalysatoren Titan- bzw. Zirkoniumacetylacetonate der allgemeinen Formel (IV)

$(R^5O)_mM(ACA)_n$ (IV)

in der $R^5$ eine Alkylgruppe mit 1 bis 4 Kohlenstoffatomen, ACA einen Acetylacetonatrest und m die Zahl 0 und n die Zahl 4 oder m die Zahl 2 und n die Zahl 2 bedeuten.

Gemäß einer weiteren vorteilhaften Ausführungsform der Erfindung verwendet man als Katalysatoren Polykondensationsprodukte von Estern der Titansäure mit Monoalkanolen mit 2 bis 10 Kohlenstoffatomen mit Pentaerythrit.

Gemäß einer weiteren vorteilhaften Ausführungsform der Erfindung verwendet man die erfindungsgemäß einzusetzenden Katalysatoren in einer Menge von 0,1 bis 10, vorzugsweise 1 bis 5, Mol-%, bezogen auf vorhandene Fettsäurereste.

Gemäß der Erfindung führt man die Kondensationsreaktion bei 150 bis 270 °C, vorzugsweise im Vakuum sowie unter Schutzgas, durch. Man kann das gebildete Reaktionswasser zusammen mit den 2-alkyl- bzw. alkenylsubstituierten 2-Oxazolinen abdestillieren und von diesen während der Destillation trennen, wobei man mitgeschlepptes Restwasser, insbesondere bei kürzerkettigen 2-Alkyl- bzw. Alkenyl-2-oxazolinen mit üblichen Trocknungsmitteln wie wasserfreiem Natriumsulfat oder Molekularsieb (4A) abtrennt. Es ist jedoch auch möglich, das Reaktionswasser durch azeotrope Destillation mittels hochsiedender Schleppmittel wie z.B. Tetralin oder Cumol aus dem Reaktionsgemisch vor der eigentlichen Destillation der 2-Oxazoline zu entfernen.

Gemäß einer weiteren bevorzugten Ausführungsform der Erfindung kann man die 2-alkyl- bzw. alkenylsubstituierten 2-Oxazoline direkt aus Fettsäureglyceriden herstellen, indem man diese in Gegenwart von Ethanolamin bei erhöhter Temperatur und bei atmosphärischem Druck zu den Fettsäureethanolamiden umsetzt, gebildetes Wasser und Glycerin sowie nicht umgesetztes bzw. überschüssiges Ethanolamin durch Destillation im Vakuum praktisch vollständig entfernt und das Reaktionsgemisch in einer zweiten Reaktionsstufe im Vakuum unter weiterer Steigerung der Reaktionstemperatur reagieren läßt. Die Glyceride reagieren in Gegenwart von Ethanolamin und den beanspruchten Katalysatoren in der ersten Reaktionsstufe zu Fettsäureethanolamiden, welche in der zweiten Reaktionsstufe zu den 2-Oxazolinen reagieren. Vorzugsweise setzt man das Ethanolamin dabei in einem 50 bis 400 mol-%-igen Überschuß, bezogen auf Ausgangsmaterial, ein. In der ersten Reaktionsstufe verwendet man bevorzugt eine Reaktionstemperatur von weniger als 185 °C und in der zweiten eine solche von 185 bis 270 °C. Die Katalysatoren werden bevorzugt in einer Menge von 0,1 bis 10, vorzugsweise 1 bis 5, mol-%, bezogen auf vorhandene Fettsäurereste, verwendet. Dabei werden wiederum bevorzugt Titantetraalkoholate, insbesondere ausgewählt von der aus Titantetraethylat, -tetrapropylat, -tetraisopropylat und -tetrabutylat gebildeten Gruppe, eingesetzt. Alternativ werden gemischte Anhydride der Titansäure mit Monocarbonsäuren, insbesondere solcher mit 1 bis 4 Kohlenstoffatomen, vorzugsweise Titantetraacetat, verwendet. Weiterhin hier bevorzugte Katalysatoren sind Titan- bzw. Zirkoniumacetylacetonate der allgemeinen Formel IV sowie die oben erwähnten Polykondensationsprodukte von Estern der Titansäure mit Monoalkanolen mit 2 bis 10 Kohlenstoffatomen mit Pentaerythrit sowie Titan (IV)- bzw. Zr(IV)-tetraaminoethanolate.

Bei dieser Verfahrensvariante führt man die zweite Stufe der Reaktion im Vakuum durch und destilliert das gebildete Reaktionswasser zusammen mit den 2-alkyl- bzw. alkenylsubstituierten 2-Oxazolinen unter weiterer Auftrennung während der Destillation. Man kann jedoch auch vor der Destillation der 2-Alkyl- bzw. Alkenyl-2-oxazoline das Reaktionswasser durch azeotrope Destillation entfernen.

Besonders vorteilhaft ist es, hier die erste und zweite Reaktionsstufe als Ein-Topf-Verfahren in einem Reaktor durchzuführen.

Die Erfindung wird im folgenden anhand von bevorzugten Ausführungsbeispielen näher erläutert.

Beispiel 1.

Herstellung von 2-Fettalkenyl-2-oxazolin aus Sojaöl.

In einer Rührapparatur mit Fraktionierkolonne, absteigendem Kühler mit Destillationsvorlagen, Vakuumeinrichtung mit Kühlfalle für das Reaktionswasser, Thermometer und Einleitungsrohr für Schutzgas wurde eine Mischung aus

295 g (0,333 mol) Sojaöl, entschleimt, Hydroxylzahl 2,3, Jodzahl 128,6, Verseifungszahl 193,8, Säurezahl 0,5,

123 g (2 mol) Ethanolamin (99,5 %-ig),

3,4 g (0,01 mol, 3 mol-%) Titan-tetrabutylat

vorgelegt.

Nach 4 Stunden Erhitzen unter Stickstoff am Rückfluß bei Normaldruck wurde im Vakuum bei 81 bis 87 °C und 20 hPa das überschüssige Ethanolamin abdestilliert. Anschließend wurde im Vakuum bei 144 bis 182 °C und 0,8 bis 0,1 hPa das entstandene Glycerin abdestilliert.

Das so erhaltene Sojafettsäureethanolamid wurde im Vakuum auf 194 bis 268 °C erhitzt, wobei bei 160 bis 194 °C und 0,07 hPa das 2-Sojafettalkenyl-2-oxazolin und Wasser gleichzeitig überdestillierten. Das Wasser wurde zum größten Teil in der Kühlfalle kondensiert. Um das überdestillierte Oxazolin von eventuell mitgerissenem Wasser zu befreien, wurde in die Destillationsvorlagen als Trockenmittel Natriumsulfat gegeben.

Man erhielt 241,6 g (79 % der Theorie, bezogen auf eingesetztes Sojaöl) an 2-Sojafettalkenyl-2-oxazolin.

IR: 1671 (C=N); 1230, 1171 (C-O-C=); 989, 953, 915 $cm^{-1}$ (Oxazolin).

Beispiel 2.

Herstellung von 2-Fettalkenyl-2-oxazolin aus Sojaöl.

Analog der Arbeitsweise des Beispiels 1 wurde raffiniertes Sojaöl mit Ethanolamin zum 2-Sojafettalkenyl-2-oxazolin umgesetzt. Die Ansatzmengen waren:

443 g (0,50 mol) Sojaöl, raffiniert, Hydroxylzahl 0,0, Jodzahl 127,5, Verseifungszahl 193,0, Säurezahl 0,2

184,2 (3 mol) Ethanolamin (99,5 %-ig)

5,1 g (0,015 mol, 1 mol-%) Titan-tetrabutylat.

Ausbeute: 351,8 g (77 % der Theorie)

Kp = 178 bis 200 °C (0,06 hPa)

IR: 1670 (C=N); 1231, 1170 (C-O-C=); 989, 953, 915 $cm^{-1}$ (Oxazolin);

$^1$H-NMR: delta (in ppm) = 5,35 (ca. 3H,m), 4,21 (2H,t,J = 7 Hz), 3,82 (2H,t,J = 6 Hz), 2,77 (ca. 1,5H,br,t,J = 5 Hz), 2,25 (2H,t,J = 6 Hz), 2,04 (4H,m), 1,63 (2H,p,J = 6 Hz), 1,29 (ca. 18H,m), 0,88 (3H,t,J = 6 Hz), 0,89 (3H,t,J = 6 Hz).

Beispiel 3.

Herstellung von 2-Talgalkyl-2-oxazolin aus gehärtetem Talg.

Analog der Herstellung zum Beispiel 1 wurde gehärteter Talg mit Ethanolamin umgesetzt.
Die Ansatzmengen waren:
428 g (0,50 mol) Rindertalg, gehärtet, handelsübliche Qualität, Jodzahl 0,1, Verseifungszahl 196,7, Säurezahl 2,8, Smp. 57,5° C, Fettsäureverteilung: ca. 28 % $C_{16}$, ca. 70 % $C_{18}$;
184,2 g (3 Mol) Ethanolamin (99,5 %-ig)
5,1 g (0,015 mol, 1 mol-%) Titan-tetrabutylat.
Ausbeute: 371,0 g (83 % der Theorie)
Kp = 188 bis 210° C (0,04 bis 0,08 hPa)
IR: 1670 (C=N); 1231, 1170 (C-O-C=); 988, 954, 915 $cm^{-1}$ (Oxazolin)
$^1$H-NMR: delta (in ppm) = 4,22 (2H,t,J = 7 Hz), 3,82 (2H,t,J = 7 Hz), 2,27 (2H,t,J = 6 Hz), 1,63 (2H,p,J = 6 Hz), 1,24 (ca. 28H,m), 0,89 (3H,t,J = 6 Hz)

Beispiel 4.

Herstellung von 2-(11-Hydroxyheptadecyl)-2-oxazolin aus hydriertem Rizinusöl.

Analog der Herstellung zum Beispiel 1 wurde ein handelsübliches hydriertes Rizinusöl mit Ethanolamin umgesetzt. Die Ansatzmengen waren:
473 g (0,5 mol) Rizinusöl, hydriert, Verseifungszahl 178,7, Säurezahl 0,2
184,2 g (3 Mol) Ethanolamin (99,5 %-ig)
15,3 g (0,045 mol, 3 mol-%) Titan-tetrabutylat.
Ausbeute = 371 g 2-(11-Hydroxyheptadecyl)-2-oxazolin.
Kp = 214 bis 223° C (0,3 bis 0,7 hPa)
IR: 3295 (OH); 1671 (C=N), 1172 (C-O-C=); 1134, 992, 962, 923 $cm^{-1}$ (Oxazolin).
$^1$H-NMR: delta (in ppm) = 5,4 (ca. 0,25 H,m), 4,22 (2H,t,J = 7Hz), 3,83 (2H,t,J = 7 Hz), 3,58 (ca. 0,75 H,m), 2,26 (2H,t,J = 6 Hz), 1,98 (ca. 0,5 H,m), 1,63 (2H,m), 1,24 (ca. 16 H,m), 0,89 (3H,t,J = 6 Hz).

Beispiel 5.

Herstellung von 2-(Sojafettalkenyl)-2-oxazolin aus raffiniertem Sojaöl.

Analog der Herstellung zum Beispiel 1 wurde raffiniertes Sojaöl mit Ethanolamin in Gegenwart von Zirkon-tetrabutylat zum 2-(11-Hydroxyheptadecyl)-2-oxazolin umgesetzt. Die Ansatzmengen waren:
434,2 g (0,5 mol) Sojaöl, entschleimt; VZ = 193,8.
184,2 g (3,0 mol) Ethanolamin, 99,5 %-ig
5,8 g (0,015 mol, 1-mol-% bezogen auf eingesetzte Fettsäurereste) Zirkon-tetrabutylat
Ausbeute = 264 g (58 % d. Th.) 2-(Sojafettalkenyl)-2-oxazolin.
Kp = 187 - 219° C (0,05 - 0,1 hPa)
Das $^1$H-NMR und IR standen im Einklang mit der Struktur.

Beispiel 6.

Herstellung von 2-(Rapsfettalkyl)-2-oxazolin aus Rapsöl.

Analog der Herstellung zum Beispiel 1 wurde Rapsöl, dessen Fettsäureanteil zu etwa 40 % aus Erucasäure besteht, mit Ethanolamin zum 2-(Rapsfettalkyl)-2-oxazolin umgesetzt. Die Ansatzmengen waren:
474,0 g (ca. 0,5 mol) Rapsöl; VZ = 175.

184,0 g (3,0 mol) Ethanolamin, 99,5 %-ig
15,3 g (0,045 mol, 3 mol-% bezogen auf eingesetzte Fettsäurereste) Titan-tetrabutylat
Ausbeute = 353 g (73 % d. Th.) 2-(Rapsfettalkyl)-2-oxazolin.
Kp = 202 - 230 °C (0,01 - 0,05 hPa)
IR: 1670 (C=N); 1231, 1171 (C-O-C=); 989, 953, 915 $cm^{-1}$ (Oxazolin).
$^1$H-NMR: delta (in ppm) 5,36 (ca. 2.5H,m); 4,20 (2H,t,J = 6 Hz); 3,71 (2H,t,J, = 6 Hz); 2,70 (<1H,m); 2,24 (2H,t,J = 6 Hz); 2,02 (ca. 4H,m); 1,63 (2H,br p,J = 6 Hz); 1,26 (ca. 24H, br s); 0,88 (3H,t,J = 5 Hz).

Beispiel 7.

Herstellung von 2-(Sojafettalkenyl)-2-oxazolin aus entschleimtem Sojaöl.

Analog der Herstellung zum Beispiel 1 wurde entschleimtes Sojaöl mit Ethanolamin in Gegenwart von Titan(IV)-diisopropoxy-bis-acetylacetonat zum 2-(Sojafettalkenyl)-2-oxazolin umgesetzt.
Die Ansatzmengen waren:
434,2 g (0,5 mol) Sojaöl, entschleimt; VZ = 193,8.
184,2 g (3,0 mol) Ethanolamin, 99,5 %-ig
5,5 g (0,015 mol, 1-mol-% bezogen auf eingesetzte Fettsäurereste)
Titan(IV)-Diisopropoxy-bis-acetylacetonat
Ausbeute = 359,4 g (80 % d. Th.) 2-(Sojafettalkenyl)-2-oxazolin.
Kp = 187 - 200 °C (0,1 hPa)
Das $^1$H-NMR und IR standen im Einklang mit der Struktur.

Beispiel 8.

Herstellung von 2-(Sojafettalkenyl)-2-oxazolin aus entschleimtem Sojaöl.

Analog der Herstellung zum Beispiel 1 wurde entschleimtes Sojaöl mit Ethanolamin in Gegenwart von Zirkonacetylacetonat zum 2-(Sojafettalkenyl)-2-oxazolin umgesetzt.
Die Ansatzmengen waren:
434,2 g (0,5 mol) Sojaöl, entschleimt; VZ = 193,8.
184,2 g (3,0 mol) Ethanolamin, 99,5 %-ig
7,3 g (0,015 mol, 1-mol-% bezogen auf eingesetzte Fettsäurereste) Zirkonacetylacetonat.
Ausbeute = 334 g (74 % d. Th.) 2-(Sojafettalkenyl)-2-oxazolin.
Kp = 234 - 212 °C (0,1 hPa)
Das $^1$H-NMR und IR standen im Einklang mit der Struktur.

## Ansprüche

1. Verfahren zur Herstellung von 2-Alkyl- bzw. Alkenyl-2-oxazolinen der allgemeinen Formel I

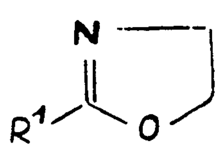

(I)

in der
$R^1$ ein gegebenenfalls hydroxy-substituierter Kohlenwasserstoffrest mit mindestens 7, insbesondere 7 bis 21 Kohlenstoffatomen in der Kohlenwasserstoffkette ist, durch Kondensation von Fettsäureethanolamiden bzw. deren Vorläufern in Gegenwart von Katalysatoren in flüssiger Phase, dadurch gekennzeichnet, daß man Glyceride, insbesondere Triglyceride, von gegebenenfalls hydroxy-substituierten Fettsäuren mit mindestens

8, insbesondere 8 bis 22 Kohlenstoffatomen mit 2-Aminoethanol
in Gegenwart von Titan- bzw. Zirkoniumverbindungen der allgemeinen Formel II
$M(OR^2)_4$ (II)
in der M vierwertiges Titan oder Zirkonium und $R^2$ eine Alkylgruppe mit mindestens 2, insbesondere 2 bis 4 Kohlenstoffatomen, eine Acylgruppe mit mindestens 2, insbesondere 2 bis 10 Kohlenstoffatomen oder eine 2-Aminoethylenoxygruppe oder einen Rest eines beta-Diketons der allgemeinen Formel III bedeuten,
$R^3$-C =CH-CO-$R^4$ (III)
in der $R^3$ und $R^4$ gleiche oder verschiedene Reste aus der von Alkylgruppen mit 1 bis 4 Kohlenstoffatomen und gegebenenfalls in p-Stellung substituiertem Phenyl gebildeten Gruppe sind, wobei jeweils zwei der Gruppen $R^2$ zusammen aus dem zweibindigen Rest eines zweiwertigen Alkohols mit 2 bis 4 Kohlenstoffatomen bestehen können,
in Gegenwart von Titanylacetylacetonat oder
in Gegenwart von Kondensationsprodukten von Titan(IV) bzw. Zirkonium(IV)-tetraalkoxylaten der allgemeinen Formel II, in der M und $R^2$ wie oben definiert sind, mit mehrfunktionellen Alkanolen, insbesondere mit 3 bis 12 Kohlenstoffatomen und 2 bis 6 Hydroxylgruppen,
kondensiert und die so erhaltenen 2-Alkyl- bzw. 2-Alkenyloxazoline unter Entfernung von gebildetem Wasser und Glycerin isoliert.

2. Verfahren nach Anspruch 1, dadurch gekennzeichnet, daß man als Katalysatoren Ester der Titansäure ($H_4TiO_4$) oder Zirkonsäure ($H_4ZrO_4$) bzw. gemischte Anhydride der Titansäure oder Zirkonsäure mit organischen Säuren der allgemeinen Formel II, in der M = Ti oder Zr und $R^2$ eine Alkylgruppe mit 2 oder mehr als 2, insbesondere 2 bis 4 Kohlenstoffatomen bzw. eine von einer Monocarbonsäure abgeleitete Acylgruppe mit 2 oder mehr als 2, insbesondere 2 bis 10 Kohlenstoffatomen bedeuten, verwendet.

3. Verfahren nach Anspruch 1 oder 2, dadurch gekennzeichnet, daß man Katalysatoren aus der von Titan- bzw. Zirkoniumtetraethylat, -tetrapropylat, -tetraisopropylat, -tetrabutylat und -tetraacetat gebildeten Gruppe verwendet.

4. Verfahren nach Anspruch 1 oder 2, dadurch gekennzeichnet, daß man Tetra(aminoethyl)-titanat oder -zirkonat als Katalysator verwendet.

5. Verfahren nach Anspruch 1 oder 2 , dadurch gekennzeichnet, daß man als Katalysatoren Titan- bzw. Zirkoniumacetylacetonate der allgemeinen Formel IV
$(R^5O)_mM(ACA)_n$ (IV)
in der $R^5$ eine Alkylgruppe mit 1 bis 4 Kohlenstoffatomen, ACA einen Acetylacetonatrest und m die Zahl 0 und n die Zahl 4 oder m die Zahl 2 und n die Zahl 2 bedeuten,
verwendet.

6. Verfahren nach Anspruch 1 oder 2, dadurch gekennzeichnet, daß man als Katalysatoren Polykondensationsprodukte von Estern der Titansäure mit Monoalkanolen mit 2 bis 10 Kohlenstoffatomen mit Pentaerythrit verwendet.

7. Verfahren nach mindestens einem der Ansprüche 1 bis 6, dadurch gekennzeichnet, daß man die Katalysatoren in Mengen von 0,1 bis 10, vorzugsweise 1 bis 5 Mol-%, bezogen auf vorhandene Fettsäurereste, verwendet.

8. Verfahren nach mindestens einem der Ansprüche 1 bis 7, dadurch gekennzeichnet, daß man in einer ersten Reaktionsstufe die Fettsäureglyceride in Gegenwart von Ethanolamin bei erhöhter Temperatur und bei atmosphärischem Druck zu den Fettsäureethanolamiden umsetzt, gebildetes Wasser und Glycerin sowie nicht umgesetztes bzw. überschüssiges Ethanolamin durch Destillation im Vakuum praktisch vollständig entfernt und das Reaktionsgemisch in einer zweiten Reaktionsstufe im Vakuum unter weiterer Steigerung der Reaktionstemperatur reagieren läßt.

9. Verfahren nach Anspruch 8, dadurch gekennzeichnet, daß man das Ethanolamin in einem 50 bis 400 Mol-%-igem Überschuß, bezogen auf in den Glyceriden vorhandene Fettsäurereste, verwendet.

10. Verfahren nach einem der Ansprüche 8 oder 9, dadurch gekennzeichnet, daß man in der ersten Reaktionsstufe eine Reaktionstemperatur von weniger als 185 °C verwendet.

11. Verfahren nach mindestens einem der Ansprüche 8 bis 10, dadurch gekennzeichnet, daß man in der zweiten Reaktionsstufe eine Reaktionstemperatur von 185 bis 270 °C verwendet.

12. Verfahren nach mindestens einem der Ansprüche 8 bis 11, dadurch gekennzeichnet, daß man in der zweiten Reaktionsstufe gebildetes 2-Oxazolin und Wasser fortlaufend abdestilliert und das Wasser aus dem Destillat abtrennt.

13. Verfahren nach mindestens einem der Ansprüche 8 bis 12, dadurch gekennzeichnet, daß man die erste und zweite Reaktionsstufe als Ein-Topf-Verfahren durchführt.

Europäisches Patentamt

# EUROPÄISCHER RECHERCHENBERICHT

Nummer der Anmeldung

EP 90 10 7492

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | CHEMICAL ABSTRACTS, Band 87, Nr. 17, 24. Oktober 1977, Seite 729, Zusammenfassung Nr. 135352h, Columbus, Ohio, US; & JP-A-77 19 661 (MITSUI PETROCHEMICAL INDUSTRIES LTD) 15-02-1977<br>* Zusammenfassung *<br>--- | 1 | C 07 D 263/12<br>C 07 D 263/14 |
| A | DE-B-1 094 749 (ROHM AND HAAS)<br>* Insgesamt *<br>--- | 1 | |
| D,A | US-A-3 681 329 (MORTON H. LITT)<br>* Patentansprüche *<br>--- | 1 | |
| P,X | EP-A-0 315 856 (HENKEL KGaA)<br>* Patentansprüche *<br>----- | 1-13 | |
| | | | RECHERCHIERTE SACHGEBIETE (Int. Cl.5) |
| | | | C 07 D 263/00 |

Der vorliegende Recherchenbericht wurde für alle Patentansprüche erstellt

| Recherchenort | Abschlußdatum der Recherche | Prüfer |
|---|---|---|
| DEN HAAG | 22-06-1990 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)